(19) Europäisches Patentamt
European Patent Office
Office européen des brevets

(11) Publication number: **0 150 832**
**B1**

(12) # EUROPEAN PATENT SPECIFICATION

(45) Date of publication of patent specification: **02.11.88**

(51) Int. Cl.⁴: **C 07 C 1/24**, C 07 C 11/02, C 07 C 13/19

(21) Application number: **85100784.9**

(22) Date of filing: **25.01.85**

(54) **A method for producing compounds having a double bond at the terminal.**

(30) Priority: **27.01.84 JP 13920/84**
**24.08.84 JP 177217/84**
**28.11.84 JP 252298/84**

(43) Date of publication of application:
**07.08.85 Bulletin 85/32**

(45) Publication of the grant of the patent:
**02.11.88 Bulletin 88/44**

(84) Designated Contracting States:
**DE FR GB IT NL**

(56) References cited:
**CH-A- 421 077**
**US-A-4 490 567**

**JOURNAL OF THE AMERICAN CHEMICAL SOCIETY, vol. 85, 1963, Easton, A.J. LUNDREN et al. "Selective catalytic dehydration of 2-alcohols; a new synthesis of 1-olefins", pp. 2180-2181**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Araki, Masashi**
**1353-4 Shiizu**
**Ichihara-shi (JP)**
Inventor: **Takahashi, Kazuteru**
**1929, Kamekubo Oimachi**
**Iruma-gun Saitama-ken (JP)**
Inventor: **Hibi, Takuo**
**135, Iriyamazu**
**Ichihara-shi (JP)**

(74) Representative: **Vossius & Partner**
**Siebertstrasse 4 P.O. Box 86 07 67**
**D-8000 München 86 (DE)**

**EP 0 150 832 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for producing a compound having a double bond at the terminal (hereinafter referred to as terminal olefin). More particularly, the present invention relates to a method for producing the terminal olefin by dehydration of a compound represented by the formula (I)

$$\begin{array}{c} \quad\; OH \\ \quad\; | \\ R-CH-CH_3 \end{array} \qquad (I)$$

wherein R is a $C_3$—$C_{20}$ hydrocarbon group which may have one or more double bonds.

It is hitherto well known that an olefin can be produced by dehydration of a compound represented by the formula (I). The details of the method can be taken, for example, from J. Am. Chem. Soc., *85,* 2180 (1963), or from Oil Chemistry, *17,* 236 (1968). It is also known from DE—A—144 362 and the corresponding CH—A—721077 that zirconium oxide can be used as a catalyst in the dehydration of secondary alcohols. However, dehydration with such usual solid acid catalyst mainly produced the internal olefin thus failing to selectively produce the terminal olefin. Thorium oxide is known as a catalyst for the selective production of the terminal olefin, but its use in industry is difficult because thorium is a radioactive element so that there occurs a serious problem of safety in handling thorium oxide as a catalyst.

The present inventors extensively studied to overcome the defects of the conventionally well-known catalyst, i.e.,

(1) the internal olefin is mainly produced and the selectivity of the terminal olefin is low,

(2) the catalyst contains a radioactive element so that there is a problem of safety,

and thereby to find a method which can produce the terminal olefin with good selectivity and safety. As a result, the present inventors found the following:

A compound having a double bond at the terminal can be produced by dehydration of a compound represented by the foregoing formule (I) at low costs as well as with good efficiency and safety by using high-purity zirconium oxide as a catalyst. The present inventors thus completed the present invention.

An object of the present invention is to provide a method for producing terminal olefins at low costs and with safety which is useful as a material for heat-resistant polymers, a comonomer for polyolefins, a raw material for detergents.

The starting material used in the method of the present invention is a compound represented by the formula (I),

$$\begin{array}{c} \quad\; OH \\ \quad\; | \\ R-CH-CH_3 \end{array} \qquad (I)$$

wherein R is a $C_3$—$C_{20}$ hydrocarbon group which may have one or more double bonds. R is not particularly limited so far as it is a $C_3$—$C_{20}$ hydrocarbon group, but it is preferably a $C_3$—$C_{10}$ hydrocarbon group and more preferably a $C_3$—$C_{10}$ saturated hydrocarbon group. Among them 4-methyl-2-pentanol and 1-cyclohexylethanol are most preferred. In the practice of the present invention with this material, the OH group and the hydrogen atom of the methyl group in the formula (I) are released in the form of water to selectively produce the terminal olefin.

Generally, zirconium compounds are produced by various methods using Zircon, Baddeleyite as a raw material, but complete removal of silicon, titanium, aluminum, iron, contained in raw ores is so difficult that it is usual for zirconium products to contain these impurities in trace amounts.

The zirconium oxide used in the present invention has to be of high purity, and it has to be substantially free of silicon and titanium. When the total content, as oxide, of these metals is 0.3 wt.% or less, a sufficient selectivity is obtained in the production of the terminal olefin, and when the total content is 0.1 wt.% or less, a more preferred selectivity is obtained.

The accompanying drawing shows the relationship between the total content of silicon dioxide and titanium dioxide and the selectivity of vinylcyclohexane.

Zirconium oxide used in the present invention is obtained by various methods, and particularly, one obtained by calcination of zirconium compounds at 300° to 1500°C is preferred. In this case, preferred examples of zirconium compound used for calcination include for example zirconium hydroxide, zirconyl hydroxide, zirconium nitrate, zirconyl nitrate, zirconyl carbonate, zirconium alkoxide. These compounds may be calcined in a state wherein they are supported on suitable carriers, or zirconium oxide after calcination may be supported on suitable carriers. Of course, it is also a preferred example to use zirconium oxide after calcination as a catalyst without using carriers. It is also possible to prepare the catalyst in the coexistence of a second component such as yttrium, if necessary.

The calcination temperature is generally 300° to 1500°C, but preferably, a temperature of 500° to 1100°C is employed. The calcination time is generally 0.1 to 50 hours, but preferably, a period of time of 1 to 10 hours is employed. Generally, the activity tends to lower as the calcination temperature becomes high, and sufficient activity comes to fail to appear when the temperature exceeds 1500°C.

Next, a method for practicing the present invention will be explained. A method of reaction is not particularly limited, but generally, a gas-phase reaction of fixed-bed form or fluidized-bed form is employed. The reaction temperature is generally 200° to 500°C, but preferably, a temperature of 300° to 400°C is employed. The reaction pressure is not particularly limited, and the reaction may be carried out at atmospheric pressure or under pressure a little higher than that. If necessary, a method may also be employed in which the starting material (I) is reacted in dilution with an inert gas such as nitrogen, etc. The reaction under a reduced pressure also gives a good result. For the feed rate of the starting material, a rate of 0.1 to 15 $hr^{-1}$, preferably 0.5 to 5 $hr^{-1}$, as expressed by LHSV, is employed.

The present invention will be explained in more detail with reference to the following examples.

Example 1

A hard glass tubular reactor of 12.5 mm in internal diameter having at the center a tubular sheath of 4 mm in external diameter for temperature measurement was packed with 12 ml of a zirconia catalyst, which had been prepared by calcination of zirconium hydroxide having a particle size of 0,7 to 1,7 mm at 1000°C for 2 hours, and externally heated in an electric furnace. To this tubular reactor were supplied 8.6 ml/hr (LHSV=0.72 $hr^{-1}$) of 4-methyl-2-pentanol and 3.2 l/min of nitrogen gas at atmospheric pressure through a gasificater heated by an electric furnace to contact with the catalyst. The reaction gas from the tubular reactor was analyzed by gas chromatography. As a result, the following results were found: Conversion of the starting material, 89%; selectivity of 4-methylpenten-1, 90%; selectivity of 4-methylpenten-2, 5%; and selectivity of methyl isobutyl ketone, 5%. The temperature of the electric furnace at that time was 363°C. The $SiO_2$ and $TiO_2$ contents of catalyst were 0.05 wt.% or less and 0.01 wt.% or less, respectively. The result is shown in Table 1.

Examples 2 to 4

Experiments with various kinds of alcohol were carried out in the same manner as in Example 1 except that the amount of catalyst used was 4 ml; the alcohol, a starting material, was supplied together with 0.4 l/min of nitrogen gas; and the zirconium oxide catalyst used was prepared by calcining zirconyl nitrate $[ZrO(NO_3)_2 \cdot 2H_2O]$ at 1000°C for 2 hours, and its $SiO_2$ and $TiO_2$ contents were 0.05 wt.% or less and 0.01 wt.% or less, respectively. The results are shown in Table 1.

Table 1

| Example | Catalyst | | | | Reaction | |
|---|---|---|---|---|---|---|
| | Material for catalyst | Calcination condition (°C x hr) | Impurity (wt.%) | | Starting material | Reaction temperature (°C) (furnace temperature) |
| | | | $SiO_2$ | $TiO_2$ | | |
| 1 | Zirconium hydroxide | 1000 x 2 | ≦0.05 | ≦0.01 | 4-Methyl-2-pentanol | 363 |
| 2 | Zirconyl nitrate, $ZrO(NO_3)_2 \cdot 2H_2O$ | 1000 x 2 | ≦0.05 | ≦0.01 | 2-Hexanol | 360 |
| 3 | Ditto | Ditto | Ditto | Ditto | 2-Octanol | 370 |
| 4 | Ditto | Ditto | Ditto | Ditto | 2-Nonyl-alcohol | 368 |

- Cont'd -

Table 1 (Cont'd)

| Example | Reaction | | | | |
|---|---|---|---|---|---|
| | Feed rate of starting material (ml/hr) | Conversion (%) | Selectivity of terminal olefin (%) | Selectivity of other olefins (%) | Selectivity of ketone (%) |
| 1 | 8.6 | 89 | 90 | 5 | 5 |
| 2 | 8 | 90 | 90 | 4 | 6 |
| 3 | 7 | 93 | 89 | 6 | 5 |
| 4 | 8 | 90 | 89 | 6 | 5 |

Examples 5 to 8

Experiments were carried out using catalysts prepared by changing the calcination temperature of zirconyl nitrate. The experimental condition was the same as in Example 1 except that 4-Methyl-2-pentanol was used as a starting material; 2 l/min of nitrogen gas was supplied at the same time; and the amount of every catalyst used was 4 ml. The results are shown in Table 2.

Table 2 (Effect of calcination temperature of catalyst)

| Example | Catalyst | | | | Reaction | |
|---|---|---|---|---|---|---|
| | Material for catalyst | Calcination condition (°C x hr) | Impurity (wt.%) | | Starting material | Reaction temperature (°C) (furnace temperature) |
| | | | $SiO_2$ | $TiO_2$ | | |
| 5 | Zirconyl nitrate, $ZrO(NO_3)_2 \cdot 2H_2O$ | 400 x 2 | $\leqq 0.05$ | $\leqq 0.01$ | 4-Methyl-2-pentanol | 285 |
| 6 | Ditto | 600 x 2 | Ditto | Ditto | Ditto | 299 |
| 7 | Ditto | 800 x 2 | Ditto | Ditto | Ditto | 314 |
| 8 | Ditto | 1000 x 2 | Ditto | Ditto | Ditto | 350 |

Table 2 (Cont'd)

| Example | Reaction | | | | |
|---|---|---|---|---|---|
| | Feed rate of material (ml/hr) | Conversion (%) | Selectivity of terminal olefin (%) | Selectivity of other olefins (%) | Selectivity of ketone (%) |
| 5 | 3 | 89 | 87 | 6 | 7 |
| 6 | 3 | 89 | 90 | 5 | 5 |
| 7 | 3 | 90 | 91 | 3 | 6 |
| 8 | 3 | 93 | 87 | 3 | 10 |

Example 9

A hard glass tubular reactor of 19 mm in internal diameter having at the center a tubular sheath of 6 mm in external diameter for temperature measurement was packed with 27 ml of a zirconia catalyst, which had been prepared by calcination of zirconium hydroxide having a particle size of 0,7 to 1,7 mm at 1000°C for 2 hours, and externally heated in an electric furnace. To this tubular reactor were supplied 28 ml/hr (LHSV=1.04 $hr^{-1}$) of 1-cyclohexylethanol and 1.5 l/min of nitrogen gas at atmospheric pressure through a gasificater heated by an electric furnace. The reaction gas from the tubular reactor was trapped and analyzed by gas chromatography. At the point when the activity of the catalyst reached a stationary state, the following results were found: Conversion of the starting material, 90%; selectivity of vinylcyclohexane, 90%, selectivity of ethylidenecyclohexane, 4.5%; and selectivity of methyl cyclohexyl ketone, 5.5%. The central temperature of the catalyst at that time was 376°C. An about 100-hour continuous run was made but with little change in the activity and selectively. The $SiO_2$ and $TiO_2$ contents of the catalyst were 0.05 wt.% or less and 0.01 wt.% or less, respectively. The results are collectively shown in Table 3.

Examples 10 to 17

Experiments were carried out with various catalysts and under various conditions according to Example 1. The results are shown in Table 3.

Table 3 Effects of catalyst (including calcination temperature) and reaction condition

| Example | Catalyst | | | | Reaction | |
|---|---|---|---|---|---|---|
| | Material for catalyst | Calcination condition (°C x hr) | Impurity (wt.%) | | Starting material | Reaction temperature*3 (°C) |
| | | | $SiO_2$ | $TiO_2$ | | |
| 9 | Zirconium hydroxide, $Zr(OH)_4$ | 1000 x 2 | ≦0.05 | ≦0.01 | CHE | 376 |
| 10 | Ditto | 360 x 2 | Ditto | Ditto | Ditto | 326 |
| 11 | Ditto | 405 x 2 | Ditto | Ditto | Ditto | 332 |
| 12 | Ditto | 510 x 2 | Ditto | Ditto | Ditto | 332 |
| 13 | Ditto | 610 x 2 | Ditto | Ditto | Ditto | 329 |
| 14 | Ditto | 800 x 2 | Ditto | Ditto | Ditto | 351 |
| 15 | Ditto | 900 x 2 | Ditto | Ditto | Ditto | 372 |
| 16 | $ZrO(NO_3)_2 \cdot 2H_2O$ | 400 x 2 | Ditto | Ditto | Ditto | 345 |
| 17 | $ZrO(NO_3)_2 \cdot 2H_2O$ supported on alumina*2 | 400 x 2 | Ditto | Ditto | Ditto | 350 |

- Cont'd -

Note: * Reaction was carried out using a Pyrex glass tubular reactor of 28 mm in internal diameter packed with 100 ml of the catalyst.

*2 Zirconyl nitrate was supported on an α-alumina in an amount of 20 wt.%, as converted to anhydrous basis, of the α-alumina.

*3 Central temperature of catalyst.

Table 3 (Cont'd)

| Example | Reaction | | | | | |
|---|---|---|---|---|---|---|
| | Feed rate of starting material LHSV ($hr^{-1}$) | Feed rate of $N_2$ (l/min) | Conversion (%) | Selectivity of terminal olefin (VCH) (%) | Selectivity of other olefins (EDC) (%) | Selectivity of ketone (MCK) (%) |
| 9 | 1.04 | 1.5 | 90 | 90 | 4.5 | 5.5 |
| 10 | 2.7 | 1.5 | 94 | 78 | 15 | 7 |
| 11 | 2.7 | 1.5 | 93 | 79 | 15 | 5 |
| 12 | 2.8 | 1.5 | 87 | 80 | 16 | 4 |
| 13 | 2.9 | 1.5 | 83 | 80 | 15 | 5 |
| 14 | 1.4 | 1.5 | 81 | 85 | 9 | 5 |
| 15 | 1.3 | 1.5 | 93 | 87 | 4 | 9 |
| 16 | 1.6 | 5.3* | 90 | 81 | 13 | 5 |
| 17 | 2.2 | 1.5 | 92 | 81 | 15 | 4 |

CHE 1-Cyclohexylethanol

VCH Vinylcyclohexane

EDC Ethylidenecyclohexane

MCK Methyl cyclohexyl ketone

Example 18

A stainless steel tubular reactor of 16 mm in internal diameter having at the center a tubular sheath of 4 mm in external diameter for temperature measurement was packed with 30 ml of zirconium oxide having a particle size of 0,7 to 1,7 mm and externally heated in an electric furnace. To this tubular reactor were supplied 29 ml/hr (LHSV=0.98 $hr^{-1}$) of 1-cyclohexylethanol and 1.4 l/min of nitrogen gas at atmospheric pressure through a gasificater heated by an electric furnace. The reaction gas from the reactor was trapped and analyzed by gas chromatography. As a result, the following results were found: Conversion of the starting material, 90%; selectivity of vinylcyclohexane, 84%; selectivity of ethylidenecyclohexane, 10%; and selectivity of methyl cyclohexyl ketone, 6%. The central temperature of the catalyst at that time was 365°C. The $SiO_2$ and $TiO_2$ contents of the catalyst were 0.05 wt.% or less and 0.01 wt.% or less, respectively. The results are collectively shown in Table 4.

Example 19

A stainless steel tubular reactor of 4 mm in internal diameter was packed with 7.4 ml of a zirconia catalyst prepared by calcination of zirconium hydroxide having a particle size of 0,7 to 1,7 mm at 500°C for 2 hours, and externally heated in an electric furnace. To this tubular reactor was supplied 54 ml/hr (LHSV=7.3 $hr^{-1}$) of 1-cyclohexylethanol at atmospheric pressure. The reaction gas from the reactor was analyzed by gas chromatography. As a result, the following results were found: Conversion of the material, 85%; selectivity of vinylcyclohexane, 81%; selectivity of ethylidenecyclohexane, 6%; and selectivity of methyl cyclohexyl ketone, 13%. The temperature of the electric furnace at that time was 409°C. The silicon and titanium contents, as converted to dioxide basis, of the zirconium oxide catalyst used in this example were 0.03 wt.% or less and 0.001 wt.% or less, respectively. The results are collectively shown in Table 4.

Example 20

A stainless steel tubular reactor of 8 mm in internal diameter was packed with 50 ml of a zirconia catalyst which had been prepared by calcination of zirconium hydroxide having a particle size of 0,7 to 1,7 mm at 400°C for 2 hours, and externally heated in an electric furnace. To this tubular reactor was supplied 90 ml/hr (LHSV=1.8 $hr^{-1}$) of 4-methyl-2-pentanol at atmospheric pressure. The reaction gas from the reactor was analyzed by gas chromatography. As a result, the following results were found: Conversion of the starting material, 90%; selectivity of 4-methyl-1-pentene, 81%; selectivity of 4-methyl-2-pentene, 10%; and selectivity of methyl isobutyl ketone, 9%. The temperature of the electric furnace at that time was 400°C. The silicon and titanium contents of the zirconia catalyst used in this example were the same as in Example 19. The results are collectively shown in Table 4.

Table 4

| Example | Catalyst | | | | Reaction | |
|---|---|---|---|---|---|---|
| | Material for catalyst | Calcination condition (°C x hr) | Impurity (wt.%) | | Starting material | Reaction temperature (°C) |
| | | | $SiO_2$ | $TiO_2$ | | |
| 18 | Zirconium oxide | Not calcined | $\leqq 0.05$ | $\leqq 0.01$ | CHE | $365^{*}$ |
| 19 | Zirconium hydroxide $Zr(OH)_4$ | 500 x 2 | $\leqq 0.03$ | $\leqq 0.001$ | Ditto | $409^{**}$ |
| 20 | Ditto | 400 x 2 | $\leqq 0.05$ | $\leqq 0.01$ | MPOH | $400^{**}$ |

- Cont'd -

Note: * Central temperature of catalyst. ** Furnace temperature.

CHE 1-Cyclohexylethanol

VCH Vinylcyclohexane

EDC Ethylidenecyclohexane

MCK Methyl cyclohexyl ketone

MPOH 4-Methyl-2-pentanol

$MPE^1$ 4-Methylpenten-1

$MPE^2$ 4-Methylpenten-2

MIBK Methyl isobutyl ketone

Table 4 (Cont'd)

| Example | Reaction | | | | | |
|---|---|---|---|---|---|---|
| | Feed rate of starting material LHSV ($hr^{-1}$) | Feed rate of $N_2$ (l/min) | Conversion (%) | Selectivity of terminal olefin (%) | Selectivity of other olefins (%) | Selectivity of ketone (%) |
| 18 | 0.98 | 1.4 | 90 | VCH 84 | EDC 10 | MCK 6 |
| 19 | 7.3 | 0 | 85 | VCH 81 | EDC 6 | MCK 13 |
| 20 | 1.8 | 0 | 90 | MPE[1] 81 | MPE[2] 10 | MIBK 9 |

Comparative Examples 1 to 3

Reaction was carried out in the same manner as in Example 18 except that the $SiO_2$ and $TiO_2$ contents of the catalyst used were different from those in Example 18. The results are shown in Table 5. A relationship between, on the one hand, the selectivity of vinylcyclohexane and, on the other hand, the $SiO_2$ and $TiO_2$ contents of the catalysts used in Example 18 and Comparative Examples 1 to 3, is as shown in Fig. 1 (the total content of $SiO_2$ and $TiO_2$ of the catalyst used in Example 18 was 0.06 wt.%).

Table 5

| Comparative example | Catalyst | | | | Reaction | | | | |
|---|---|---|---|---|---|---|---|---|---|
| | Material for catalyst | Calcination condition (°C x hr) | Impurity (wt.%) | | Starting material | Reaction temperature (°C)* | Feed rate of starting material LHSV ($hr^{-1}$) | Conversion (%) | Selectivity of terminal olefin(VCH) (%) |
| | | | $SiO_2$ | $TiO_2$ | | | | | |
| 1 | Zirconium oxide $ZrO_2$ | Not calcined | 0.30 | 0.08 | CHE | 381 | 0.63 | 95 | 60 |
| 2 | Ditto | Ditto | 0.20 | 0.30 | Ditto | 319 | 0.98 | 92 | 50 |
| 3 | Ditto | Ditto | 0.30 | 0.30 | Ditto | 324 | 0.98 | 93 | 43 |

Note: * Central temperature of the catalyst.

CHE 1-Cyclohexylethanol

VCH Vinylcyclohexane

**Claims**

1. A method for producing a compound having a terminally located double bond in which zirconium oxide is used as a catalyst characterized in that a compound represented by the general formula (I)

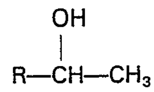

(I)

wherein R is a $C_3$—$C_{20}$ hydrocarbon group which may have one or more double bonds, is dehydrated by contacting it with a high purity zirconium oxide catalyst in which the total content of silicon and titanium, expressed as dioxides, is 0.3% by weight or less of said catalyst.

2. A method according to Claim 1, wherein the total content of silicon and titanium converted to dioxide basis is 0.1% by weight or less in the high purity zirconium oxide.

3. A method according to Claim 1, wherein the zirconium oxide is one prepared by the calcination of a zirconium compound at 300° to 1500°C.

4. A method according to Claim 3, wherein the zirconium oxide is one prepared by the calcination of a zirconium compound at 500° to 1000°C.

5. A method according to Claim 1, wherein the $C_3$—$C_{20}$ hydrocarbon group in the general formula (I) is a $C_3$—$C_{10}$ saturated hydrocarbon group.

6. A method according to Claim 5, wherein the compound of the general formula (I) is 4-methyl-2-pentanol or 1-cyclohexylethanol.

7. A method according to Claim 1, wherein the dehydration is conducted at 200° to 500°C.

8. A method according to Claim 7, wherein the dehydration is conducted at 300° to 400°C.

**Patentansprüche**

1. Verfahren zur Herstellung einer Verbindung mit endständiger Doppelbindung, in dem Zirkoniumoxid als Katalysator verwendet wird, dadurch gekennzeichnet, daß man eine Verbindung der allgemeinen Formel (I):

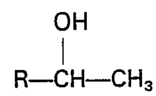

(I)

in der R ein Kohlenwasserstoffrest mit 3 bis 20 Kohlenstoffatomen ist, der eine oder mehrere Doppelbindungen aufweisen kann, durch in Kontakt-bringen mit einem hochreinen Zirkoniumoxidkatalysator, in dem der Gesamtgehalt an Silicium und Titan, in Form von Dioxiden, 0,3 Gew.-% oder weniger des Katalysators beträgt, dehydratisiert.

2. Verfahren nach Anspruch 1, bei dem in dem hochreinen Zirkoniumoxid der Gesamtgehalt an Silicium und Titan, umgerechnet auf Dioxidbasis, 0,1 Gew.-% oder weniger beträgt.

3, Verfahren nach Anspruch 1, bei dem das Zirkoniumoxid durch Kalzinieren einer Zirkoniumverbindung bei 300° bis 1500°C hergestellt wurde.

4. Verfahren nach Anspruch 3, bei dem das Zirkoniumoxid durch Kalzinieren einer Zirkoniumverbindung bei 500° bis 1000°C hergestellt wurde.

5. Verfahren nach Anspruch 1, bei dem der Kohlenwasserstoffrest mit 3 bis 20 Kohlenstoffatomen der allgemeinen Formel (I) ein gesättigter Kohlenwasserstoffrest mit 3 bis 10 Kohlenstoffatomen ist.

6. Verfahren nach Anspruch 5, bei dem die Verbindung der allgemeinen Formel (I) 4-Methyl-2-pentanol oder 1-Cyclohexyläthanol ist.

7. Verfahren nach Anspruch 1, bei dem die Dehydratisierung bei 200° bis 500°C durchgeführt wird.

8. Verfahren nach Anspruch 7, bei dem die Dehydratisierung bei 300° bis 400°C durchgeführt wird.

**Revendications**

1. Procédé de préparation d'un composé ayant une double liaison terminale, suivant lequel on utilise de l'oxyde de zirconium comme catalyseur, caractérisé en ce qu'un composé, représente par la formule générale (I)

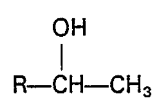

(I)

dans laquelle R est un groupe hydrocarboné en $C_3$—$C_{20}$ pouvant contenir une ou plusieurs doubles liaisons, est déshydraté par mise en contact avec un catalyseur qui est de l'oxyde de zirconium de haute

pureté, dans lequel la teneur totale en silice et en titane, exprimée en dioxydes, est de 0,3% en poids au maximum par rapport audit catalyseur.

2. Procédé selon la revendication 1, suivant lequel la teneur totale de l'oxyde de zirconium de haute pureté en silice et en titane, exprimée en dioxydes, est de 0,1% en poids au maximum.

3. Procédé selon la revendication 1, dans lequel l'oxyde de zirconium est préparé par calcination d'un composé de zirconium à une température de 300° à 1500°C.

4. Procédé selon la revendication 3, dans lequel l'oxyde de zirconium est préparé par calcination d'un composé de zirconium à une température de 500° à 1000°C.

5. Procédé selon la revendication 1, suivant lequel le groupe hydrocarboné en $C_3$—$C_{20}$ dans la formule générale (I) est un groupe hydrocarboné saturé en $C_3$—$C_{20}$.

6. Procédé selon la revendication 5, dans lequel le composé de la formule générale (I) est le 4-méthyl-2-pentanol ou le 1-cyclohexyléthanol.

7. Procédé selon la revendication 1, dans lequel la déshydratation est effectuée à une température de 200° à 500°C.

8. Procédé selon la revendication 7, dans lequel la déshydratation est effectuée à une température de 300°C à 400°C.

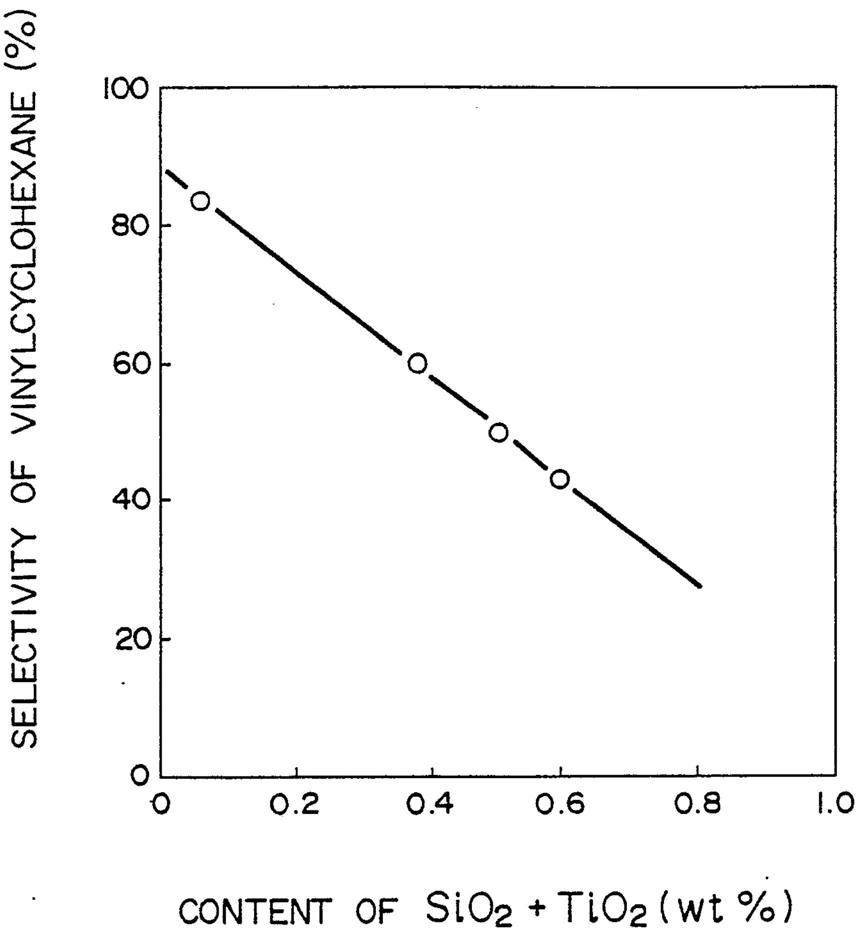
SELECTIVITY OF VINYLCYCLOHEXANE (%)
100
80
60
40
20
0
0
0.2
0.4
0.6
0.8
1.0
CONTENT OF $SiO_2 + TiO_2$ (wt %)